# EUROPEAN PATENT APPLICATION

(11) **EP 1 522 585 A1**
(43) Date of publication of application: **13.04.2005**
(21) Application number: 03078192.6
(22) Date of filing: 09.10.2003
(51) Int. Cl.: C12N 15/82, C07K 14/28

(54) **Chimeric carrier molecules for the production of mucosal vaccines**

(71) Applicant: Plant Research International B.V., 6708 PB Wageningen (NL)
(72) Inventor: Florack, Dionisius Elisabeth Antonius, 6708 MD Wageningen (NL); Bosch, Hendrik Jan, 6708 NA Wageningen (NL)
(74) Representative: Prins, Adrianus Willem, Mr. Ir.

(57) **Abstract**

The invention relates to a protein complex for the delivery of an antigen to and across mucosal surfaces and the production of said complex in a host cell, such as a plant. Provided is a protein complex comprising at least two, preferably identical, subunits wherein at least one subunit is unaltered and at least one subunit is fused to a first molecule of interest and wherein the protein complex is able to interact with a cell surface receptor via said subunits. Also provided is a method for producing a protein complex according to the invention, comprising a) providing a host cell with a nucleotide sequence encoding an unaltered subunit and a nucleotide sequence encoding a molecule of interest, wherein at least one molecule of interest is fused to a subunit; b) culturing said host cell thereby allowing expression of said nucleotide sequences and allowing for assembly of the protein complex; c) isolating the complex; d) determining the binding of the complex to a cell surface receptor or to a molecule which mimics a cell surface receptor.

## Description

The invention relates to the development, composition and the production of mucosal (e.g. oral or nasal) vaccines. More specifically, the invention relates to a protein complex for the delivery of an antigen to and across mucosal surfaces and the production of said complex in a host cell, such as a plant.

Oral vaccination is regarded to be an attractive alternative for injected vaccines because an oral vaccine is, generally speaking, easy to apply and relatively cheap and safe. More important, it can induce protection at the mucosal level, i.e. at the site of entrance of many pathogens. Vaccine administration at a mucosal site, for example by oral or nasal delivery, may even be a prerequisite for the production of vaccines against certain pathogens for which no vaccine is currently available (e.g. respiratory syncytial virus and even possibly HIV). In addition, oral vaccination enables mass vaccination via food or drinking water.

However, oral vaccination often appears not to be very effective. The immune response is short-lasting and typically large doses of antigen are required to elicit the desired effect, even when alive micro-organisms are used. This is due in part to inefficient uptake of antigen (Lavell et al. Adv Drug Delivery Rev 1995; 18:5-22). As a result, various strategies for effective delivery of antigens by mucosal routes have been investigated (see for example O'Hagan, J Pharm Pharmacol (1997)49:1-10; Husband, Vaccine (1193) 11:107-112). Antigens which are successfully delivered across the barrier of epithelial cells lining mucosal tracts stimulate underlying inductive sites of the mucosa-associated lymphoid tissue (MALT). Antigen-specific lymphocytes which are sensitised in the MALT migrate through the circulatory system to populate distant mucosal sites. Thus, mucosal immunisation immunisation may provide both local and systemic protection.

One strategy to develop mucosal vaccines has been to employ bacterial enterotoxins such as cholera toxin (CT) from *Vibrio cholera* and the related *E*. *coli* heat-labile enterotoxin (LT), which are highly immunogenic when delivered mucosally and which can act as carrier molecules and adjuvant to potentiate responses to non-related antigens, the latter especially when the holotoxin is used.
The heat-labile enterotoxin of enterotoxigenic *Escherichia coli* (LT) and related cholera toxin (CT) from *Vibrio cholerae* are extremely potent immunogens and mucosal adjuvants upon oral administration (for review see Spangler, 1992 and Williams *et al.,* 1999). Both toxins comprise an A subunit and a pentameric ring of identical B subunits. The A subunit is the toxic part of the chimaeric molecule and causes ADP-ribosylation of G_{sα} activating adenylate cyclase leading to elevation of cyclic AMP levels. This ultimately results in water loss into the gut lumen characterized by watery diarrhoea. The primary function of the strong non-covalently associated complex comprising the pentameric B subunit is in mediating receptor interactions that result in internalisation and uptake of the toxic A subunit. The primary receptor of the ring of B subunits is the monosialoganglioside GM1 [Gal(β1-3)GalNAcβ(1-4)(NeuAc(α2-3))Galβ(1-4)Glc(β1-1) ceramide], a glycosphingolipid found ubiquitously on the cell surface of mammalian cells including small intestine (Holmgren, 1973; Holmgren *et al.,* 1973, 1975). This unique feature makes the B subunit crucial with respect to triggering the key immunomodulatory events associated with adjuvant activity. It also turns the B subunit into a powerful so-called mucosal carrier molecule. A mucosal carrier molecule is a molecule that interacts, e.g. via a receptor, with immuno-active cells located on the surface of mucosae, such as the mucosa of intestinal epithelium of the small intestine.

The B subunits of both LT (LTB) and CT (CTB) have been successfully used as mucosal carrier molecule in translational fusions with diverse antigens to shuttle these across the gut mucosal epithelium by receptor-mediated uptake (e.g. Dertzbaugh and Elson, 1993; Jagusztyn-Krynicka et al., 1993). Presumably, fusion of an antigen to the carrier molecule enhances the amount of antigen delivered to the MALT inductive sites and the subsequent stimulation of antigen-specific B- and T-lymphocytes. Antigen can also be chemically coupled to LTB (O'Dowd et al. Vaccine (1999) 17:1442-1453; Green et al. Vaccine (1996)14:949-958). In addition, both LTB and CTB also improved immune responses upon oral uptake when co-administered along with antigens (Bowen et al., 1994; Wilson et al., 1993).

Whereas genetic fusion of antigens or epitopes to LTB or CTB has been successful in some cases, it appeared that translational fusion of heterologous epitopes to the B subunits can interfere with the structure, secretion, GM1-binding and immunogenicity of the LTB or CTB fusion proteins, as reported for example by Sandkvist et al (J Bacteriol 1987 169:4570), Schodel et al. (Gene 1991;99:255) and Dertzbaugh et al. (Infect Immun 1993; 61:384). Apparently, there are limitations to the size and type of antigen which can be attached to LTB or CTB such that pentamerization and GM1 binding are retained. For the development of an LTB- or CTB-based vaccine this limitation is especially relevant since most functional vaccines are composed of large structural proteins. In addition, many protective antigens (viral, bacterial and others) are composed of multimeric complexes, either homomultimeric or heteromultimeric, and only induce a protective immune response when delivered as such. For example, a classical swine fever (CSFV) E2 glycoprotein-based vaccine contains a CSFV-E2 homodimer. The multimeric nature of various protective antigens greatly reduces the use of LTB and/or CTB as carrier molecule using conventional genetic fusions of these types of antigens, since in this way a complex of five identical fusion proteins is formed.

For commercialisation and market introduction, large-scale production of the fusion protein is required and the product needs to be safe and nearly pure. Production of such vaccines based on fusion proteins of LTB and CTB and antigens, in bacteria and yeast requires large-scale fermentation technology and stringent purification protocols to obtain sufficient amounts of recombinant protein for oral delivery. Transgenic plants and especially edible plant parts, are safe expression systems for vaccines for oral delivery (for review see Langridge, 2000; Mason and Arntzen, 1995). Recently, LTB and CTB were successfully produced in diverse plant species, including tobacco, potato and corn (Arakawa et al., 1997; Haq et al., 1995; Hein et al., 1995; Lauterslager et al., 2001; Streatfield et al., 2001). Surprisingly, also plants appeared able to produce pentamers of the B subunits similar to *E. coli* and *V. cholerae.* Several groups have reported that oral immunisation of mice by means of feeding agave, potato tubers or corn accumulating either LTB or CTB resulted in both serum IgG and secretory sIgA responses (Arakawa et al., 1998; Haq et al., 1995; Lauterslager et al., 2001; Mason et al., 1998; Streatfield et al., 2001). A pre-clinical trial with human volunteers fed 50 to 100 grams amounts of transgenic tubers accumulating LTB also resulted in specific serum IgG and secretory sIgA responses (Tacket et al., 1998). Accordingly, transgenic host cell systems are in essence advantageously used for the (large-scale) production of mucosal carrier molecules such as a LTB- or CTB-fusion protein. However, it has been shown that the expression level of a fusion protein significantly decreases with increasing size and complexity of the fusion protein. For example, expression of the gene construct encoding LTB fused to the CSFV-E2 glycoprotein (approximately 35 kDa and protective as a dimer of appro 70 kDa) in potato tubers was more than 100 times lower than that of the gene encoding the LTB subunit of∼15 kDa (Lauterslager, 2002; PhD thesis University of Utrecht). Possibly, the low expression levels of large LTB- or CTB-fusion proteins fundamentally reflect instability of the large pentameric protein complex because it is often observed that monomers are more prone to (enzymatic) degradation than assembled multimeric structures.

The invention now provides the insight that the assembly, functionality and stability of a multisubunit carrier molecule is enhanced, if not all but only some of the subunits are fused to an antigen or other molecule of interest. Provided is a protein complex comprising at least two subunits wherein at least one subunit is unaltered and at least one subunit is fused to a first molecule of interest and wherein the protein complex is able to bind to a cell surface receptor.

In a preferred embodiment, said protein complex is a carrier molecule that can be used to carry or transport a molecule of interest. For example, a protein complex of the invention is a carrier molecule for the delivery of a molecule of interest to a site of interest, e.g. to and/or across the epithelial surfaces. According to the invention, a molecule of interest can comprise a variety of different classes of proteins or polypeptides or stretches thereof, that one may wish to deliver at, distribute within, transport to or retain at a certain site in an organism using the carrier properties of the complex. It especially refers to the moiety fused or added to a subunit that, when one would try to attach it to all subunits of the carrier, would interfere with the formation of a stable and functional multimeric structure of a carrier molecule e.g. by sterical hindrance. For instance, the size of said molecule of interest fused to a subunit is a quarter, a third, half, once, twice, three or even four times the size of the subunit alone.

The term "unaltered" as used herein refers to a subunit or monomer that is not fused to a molecule of interest. It is however not limited to the native subunit or monomer; for example it is a recombinant protein wherein one or more amino acids are removed from, replaced in or added to the native subunit. This may be done to modulate the production process, e.g. expression or secretion, of the recombinant protein in a (eukaryotic) host cell. For instance, an unaltered subunit may comprises a subunit provided with a signal peptide or a KDEL sequence at the C-terminus for retention in the ER. The term "unaltered subunit" essentially refers to a native subunit or a slightly modified version thereof wherein the modification does not interfere with multimerization.

In one aspect of the invention, a protein complex comprises at least two, preferably identical, subunits characterised in that at least one subunit is unaltered and at least one subunit is fused to a first molecule of interest and wherein said first molecule of interest can associate with a second molecule of interest to form a multimer of interest, and wherein the protein complex is able to interact with a cell surface receptor via said subunits. Preferably, said first molecule associates or interacts with said second molecule via an intermolecular covalent bond, for instance via one or more disulfide bridge(s). A multimer of interest according to the invention is for example a multimeric protective antigen such as a homodimeric or a heterodimeric antigen. Known multimeric protective antigens include the CSFV-E2 homodimer, the trimeric glycoprotein G of viral haemorrhagic septicaemia virus (VHSV-G) (Lorenzen, N., Lorenzen, E., Einer-Jensen, K., Heppell, J., Wu, T., Davis, H. (1998) Protective immunity to VHS in rainbow trout *(Oncorhynchus mykiss,* Walbaum) following DNA vaccination. *Fish & Shellfish Immunology* 8: 261-270; Lorenzen, N., Olesen, N.J. (1997) Immunisation with viral antigens: viral haemorrhagic septicaemia. In: Fish Vaccinology. Gudding, R., Lillehaug, A., Midlyng, P.J., Brown, F. (eds). Dev. Biol. Stand. Basel, Karger, vol. 90, p 201-209) and trimeric glycoprotein G of Rabies virus (RV G) and vesicular stomatitis virus (VSV G); the homotetrameric phosphoprotein P of Sendai virus (SeV P).

According to the invention, a second molecule of interest may be fused to a subunit. For example, in one embodiment of the invention a protein complex comprises three subunits, two of which are fused to an identical (e.g. CSFV-E2) monomer, and one unaltered subunit. It is desired that the subunits are in close proximity of each other such that the fused monomers can interact and are capable of forming a homomultimer, for instance an antigenic (e.g. CSFV-E2) homodimer. The presence of an unaltered subunit enhances the stability of the multisubunit structure of the protein complex and ensures that the conformation of the complex is retained such that it can interact via the subunits with its receptor. Obviously, it is also possible that a protein complex of at least three subunits comprises two or more subunits that are fused to different monomers and at least one unaltered monomer, such that the fused monomers can form a heteromultimer.

Of course, a second molecule of interest does not need to be fused to a subunit in order to interact with a first molecule of interest and form a multimer of interest. Moreover, according to the present invention, it may sometimes be advantageous e.g. with respect to steric hindrance, to design a protein complex comprising a multimer of interest wherein said multimer of interest is composed out of multiple molecules of interest that are not all fused to a subunit. For example, the degree of interaction between molecules of interest which are all fused to a subunit is to a certain extent determined by the relative orientation of the individual subunits they are fused to. Accordingly, molecules that are normally (i.e. in their native, non-fused conformation) capable of interacting with each other may become spatially restricted when fused to a subunit resulting in a decrease or even complete loss of interaction between the molecules. Thus, according to the present invention a protein complex may comprise a multimer of interest composed of multiple molecules of interest capable of forming a multimeric structure, for instance through disulfide bridges. Advantageously, a protein complex of the invention comprising at least one unaltered subunit and at least one subunit fused to an antigen typically displays improved folding of one or more (multimeric) antigens into an antigenic moiety. As said before, one, some, or all molecules of interest are fused to a subunit. Based on the quaternary structure and symmetry of the subunits and of multimer of interest, a person skilled in the art will be able to select the optimal number of fused versus non-fused molecules in order to obtain a protein complex comprising a multimer of interest with an optimal configuration, e.g. optimal antigenic properties.

In one embodiment, a protein complex is able to bind to a cell surface receptor that is present on intestinal epithelial, for example to a ganglioside molecule like GM1. Such a protein complex comprising a molecule of interest is advantageously used as a mucosal carrier molecule.

In a preferred embodiment, a protein complex of the invention is essentially based on the heat labile enterotoxin (LT) of *E*. *coli* or on the cholera toxin (CT) of *Vibrio cholerae,* preferably on the B subunits thereof. A complex of the invention is for instance a ring structure composed of five B subunits of LT or CT, wherein at least one subunit is unaltered and at least one subunit is fused to a molecule of interest. Such a chimeric or hetero-pentameric ring structure of the invention comprises one, two, three or four unaltered LTB- or CTB-monomers and four, three, two or one L/CTB-fusion protein, respectively. In a preferred embodiment, a molecule of interest is fused to a subunit such that it is located at the opposite side of the molecule from the receptor-binding pocket, or at least not interfering with receptor binding properties of said subunit. For example, for a complex of the invention that is based on the pentameric LTB- or CTB-structure, this means that a molecule of interest is preferably fused to the C-terminus of the B subunit (Sixma et al. 1991 Nature 351; 371-377). Following this strategy of the invention, chimeric pentamers are obtained which have retained their conformational integrity. Since LTB subunits and CTB subunits are homologous, they can be used to form a protein complex composed of a chimeric LTB/CTB-pentamer wherein at least one subunit (be it LTB or CTB) is fused to a molecule of interest and wherein at least one subunit is unaltered. LTB- and/or CTB-based chimeric protein complexes of the invention show improved binding to the GM1 receptor and increased expression levels when compared to a homopentameric structure wherein all five subunits are fused to a molecule of interest.

In another embodiment, a protein complex of the invention is based on horseradish peroxidase (HRP). A complex of the invention is for instance a structure or composition comprised of six subunits of HRP wherein at least one subunit is unaltered and at least one subunit is fused to a molecule of interest. Such a chimeric or hetero-hexameric structure of the invention comprises one, two, three, four or five unaltered HRP-subunits (monomers) and five, four, three, two or one HRP-fusion proteins, respectively.

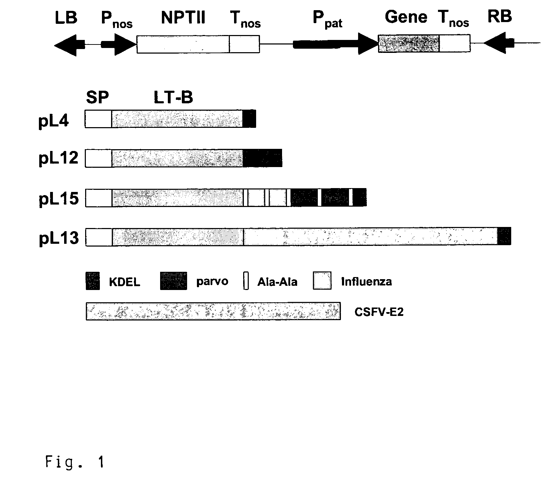

A complex according to the invention allows for optimal folding of and intramolecular interaction (e.g. via a disulphide bridge) within a multimeric antigen and thus has optimal immunogenic properties.
In one embodiment, a molecule of interest is an antigen. An antigen is any substance that stimulates the immune system. Antigens are often foreign microorganisms such as bacteria or viruses that invade the body or components of said microorganism such as proteins or protein fragments. A molecule of interest is preferably selected from the group consisting of a bacterial antigen, a viral antigen, a protozoal antigen, a nematode antigen and a fungal antigen. The invention provides a protein complex for the delivery of various antigens, for instance T- and/or B-cell epitopes such as the linear B cell epitope CPV (canine parvo virus epitope) and the T-cell specific epitope HA (influenza virus hemagglutinin epitope), and a composition comprising such a protein complex. A complex of the invention may also be used for the delivery of large antigens like for instance a viral (glyco)protein such as the E2 protein of CSFV (classical swine fever virus). One subunit fusion protein may also be fused to multiple molecules of interest. For example, a protein complex is provided comprising at least one unaltered subunit and at least one subunit translationally fused to two HA epitopes and two parvo epitopes (see Fig.1). In one embodiment of the invention, a protein complex (e.g. an LTB-based complex) comprises at least one subunit which is unaltered and at least one, preferably two, subunits which are fused to the CSFV E2 glycoprotein. In another embodiment, a protein complex of the invention comprises at least one unaltered subunit, at least one subunit which is fused to the parvo epitope and at least one subunit which is fused to multiple molecules of interest, the latter comprising a subunit fused to two HA epitopes and two parvo epitopes.

In a further embodiment, a protein complex of the invention comprises at least one subunit which is unaltered and at least one subunit which is fused to an immunomodulatory molecule (or a part thereof), preferably a protein for example a cytokine or a heat-shockprotein (HSP). A cytokine is the general term for a large group of molecules involved in signalling between cells during an immune response. Cytokines are proteins or polypeptides, some with sugar molecules attached (glycoproteins). Different groups of cytokines can be distinguished: the interferons (IFN alpha, beta, gamma); the interleukins (IL-1 to IL-15); the colony stimulating factors (CSFs) and other cytokines such as tumour necrosis factor (TNF) alpha and beta or transforming growth factor (TGF) beta. HSPs have remarkable immunomodulatory properties which derive from their interaction with macrophage and dendritic cells through a receptor, identified as CD91. For example, HSP70-2 is an important immunomodulatory protein induced in response to inflammatory stimuli. HSPs of interest to include in a protein complex according to the invention comprise HSP-60, HSP-70, HSP-90 and Gp-96. Depending on the type of the desired immunological response (e.g. Th1 versus Th2, antibody response, anti-inflammatory response), one or more (different) immunomodulatory protein(s) or part(s) thereof may be fused to a subunit of a protein complex of the invention. For example, a multicomponent vaccine of the invention comprises a protein complex comprising an antigen and an immunomodulatory molecule, preferably a cytokine, which directs an antibody response (T, B cell). On the other hand, for the treatment or prevention of for example an autoimmune disease (e.g. diabetes, multiple sclerosis) it may be advantageous to use a multicomponent vaccine or a vaccine comprising an auto-antigen and an immunomodulatory protein which directs tolerance.

Other molecules of interest comprise those molecules which, when being part of a complex of the invention, can be used as "reporter" molecule to report the location of a carrier complex within a body. This is among other helpful to monitor the *in vivo* binding of a complex to a receptor molecule and the (receptor-mediated) transport of the complex across mucosal epithelium. A reporter molecule of interest is for instance an enzyme (chloramphenicol transacetylase (CAT), neomycin phosphotransferase (neo), beta-glucuronidase (GUS) or firefly luciferase; etc.) or a fluorescent protein such as Green Fluorescent Protein (GFP) or a spectral variant thereof.

In one aspect of the invention, a protein complex comprises at least three subunits wherein at least two subunits are provided with a molecule of interest. Said at least two subunits can be translationally fused to the same molecule of interest or to a different molecule of interest. A protein complex of the invention with least two different molecules of interest is advantageously used as a carrier molecule for at least two different antigens, e.g. for the production of a multicomponent vaccine. Other combinations of different types of molecules of interest, for instance one or more antigens with one or more immunomodulatory (either stimulatory or inhibitory) proteins are of course also possible.

A cholera toxin-based multicomponent vaccine was described by Yu and Langridge (Nature, 2001, vol. 19:548), who studied the expression of a cholera toxin B subunit fused to a 22-amino acid immunodominant epitope of the murine rotavirus enterotoxin NSP4, and the ETEC fimbrial colonization factor CFA/I fused to the CTA2 subunit. Unlike in a complex of the invention, all of the subunits (both B and A2) of the reported cholera toxin complex were fused to an antigen. The fact that in this specific case no problems were encountered with CTB expression nor with the formation of a functional CTB/A2 complex was probably related to the very small size (approximately 5 kDa) of the NSP4 epitope fused to the B subunit. As said, problems with functional pentamer formation are largely due to steric hindrance and such a small epitope fused to a subunit is unlikely to interfere with pentamer formation. According to the invention, it is now also possible to produce (multicomponent) vaccines, wherein the antigens are considerably larger than 5 kDa. For example, a protein complex is provided comprising at least one (CT/LT) B subunit fused to molecule of interest, at least one unaltered B subunit and an A2 subunit fused to a different molecule of interest. Preferably, said molecule fused to the B subunit is larger than 7 kDa, more preferred larger than 10 kDa, even more preferred larger than 15 or even 25 kDa. However, in another embodiment of the invention, an (LT/CT) B subunit and an (LT/CT) A2 subunit of the same carrier complex are fused to identical antigens wherein said antigen is part of a multimeric (dimer, trimer,tetramer) protein complex with immunoprotective properties.

The invention further provides a method for producing a protein complex according to the invention, said method comprising : a) providing a host cell with a first nucleotide sequence encoding an unaltered subunit and a second nucleotide sequence encoding a subunit fused to a molecule of interest; b) culturing said host cell thereby allowing expression of said first and second nucleotide sequences and allowing for assembly of the protein complex; c) isolating the complex; and d) determining the binding of the complex to a cell surface receptor.

The term "host cell" refers to any cell capable of replicating and/or transcribing and/or translating a heterologous gene. In one embodiment, a host cell is a plant cell, a phage or a bacterium. In a preferred embodiment, a host cell of the invention is an edible host cell, which does not cause any harmful effects when consumed. Preferred examples are potato, tomato, tobacco, maize and Lactobacillus. Thus, a host cell refers to any eukaryotic or prokaryotic cell (e.g. bacterial cells such as *Escherichia coli,* yeast cells such as *Pichia pastoris,* mammalian cells such as Chinese Hamster Ovary cells, avian cells, amphibian cells, plant cells, fish cells, fungal cells such as *Agaricus bisporis,* and insect cells such as *Spodoptera frugiperda),* whether located *in vitro* or *in vivo.* For example, host cells may be located in a transgenic plant.

As described above, in a specific embodiment, a plant can be used in the method of the present invention. Said plant host maybe a monocot, such as *Zea mays, Triticum aestiuum, Oryza sativa* or *Lemna* spp., or a dicot, such as plants related to the genus *Nicotiana,* such as *N. tabacum, Lycopersicon,* such as tomato, the family *Leguminosae,* including the genus *Medicago,* or mosses such as *Physcomitrella patens.* In a preferred embodiment said host plant is *Solanum tuberosum.*

According to the invention, a host cell is provided with at least two different nucleic acid sequences: one encoding an unaltered subunit (not fused to an antigen of interest) and one encoding a subunit translationally fused to a molecule of interest. For example, a (plant) host cell is transfected with a first nucleotide construct encoding an unaltered LTB subunit and a second construct encoding an LTB-subunit fused to an antigen of interest. A nucleotide sequence encoding a subunit fused to a molecule of interest may also comprise a linker or hinge region in between the subunit and the molecule of interest to increase the flexibility of the resulting fusion protein. Following transfection and culturing under suitable conditions, said cell will express the two polypeptides to assemble a functional LTB-based chimeric protein complex "loaded" with antigen. To produce a protein complex of the invention with at least two different molecules of interest, a host cell is of course provided with at least three different nucleotide sequences. These different nucleic acid sequences can be introduced into a host cell by co-transformation of said host cell with different vectors (e.g. using T-DNA), each carrying a different nucleotide sequence. Alternatively, two or more different gene constructs can be introduced in one host cell by crossing or by using two or more expression cassettes on one binary vector. Furthermore, an established host cell line already comprising one (or more) of the components of a protein complex according to the invention can be provided with an additional nucleic acid sequence using re-transformation. In yet an alternative embodiment, a host cell expressing at least two different nucleic acid sequences is obtained by the transient (virus-mediated) expression of one sequence in a host cell which stably expresses another sequence.

An advantage of using different vectors can be sought in the fact that it allows for providing a host cell with different nucleic acid constructs in varying ratios. Herewith, it is possible to titrate the amount of unaltered, "free" subunit relative to the amount of fused subunit that is expressed by a host cell and to optimize the composition of the resulting multimeric protein complex. For instance, if a complex is desired which contains predominantly fused subunits, a host cell is co-transfected or co-transformed with construct A (unaltered subunit) and construct B (fused subunit) wherein construct B is in excess of construct A (e.g. A:B=1:3, or 1:5 or even 1:10). In contrast, excess of construct A over construct B is preferably used to increase the changes of an assembled protein complex comprising relatively few fused subunits. The latter is of course preferred if one wants to minimize negative steric effects or interference of the fused subunit with the assembly of a functional protein complex of the invention.

In yet another embodiment, a host cell is transformed with a nucleic acid construct encoding a subunit fused to a molecule of interest, e.g. LTB-CSFV E2, wherein said construct comprises a proteolytic cleavage site in between the nucleic acid sequence encoding the subunit and the nucleic acid sequence encoding the molecule of interest. Upon the partial *in vivo* cleavage of such a fusion protein by a protease (expressed endogenous of heterologous in the host cell), the host cell will contain both unaltered subunits as well as subunits fused to a molecule of interest which can form a chimeric protein complex according to the invention.

Various procedures known in the art can be used to provide a host cell with a recombinant or isolated nucleic acid (DNA or RNA). These include transformation, transfection (e.g. using calcium phosphate precipitation or a cationic liposome reagent), electroporation, particle bombardment and Agrobacterium-mediated T-DNA transfer. Depending on the type of host cell, a person skilled in the art will recognize which procedure to choose.

Following providing a host cell with said foreign nucleotides, the host cell is cultured to allow (co-)expression of said first and second nucleotide sequences and assembly of the resulting polypeptides into a protein complex of the invention. In some cases, especially when transformation or transfection procedures are relatively inefficient, it is advantageous to select those host cells which have truly received said nucleotides and to only culture those selected host cells. Host cell selection following transformation or transfection (or other procedures to provide a host cell with an isolated nucleic acid) can be performed according to standard methods. For example, most common vectors used to deliver a DNA sequence of interest to a host also contain a nucleic acid sequence encoding a protein (such as an enzyme) which, upon efficient delivery to and expression by the host cell, provides said host cell with resistance to a selection agent. A frequently used selection agent is an antibiotic, such as neomycin, kanamycin, ampicillin, carbenicillin, etc.

In a method of the invention, a (selected) host cell provided with at least two different nucleic acids (be it using a binary vector or different vectors) will express at least two different polypeptides, e.g. an unaltered (non-fused) subunit X and a fused subunit Y. Assembly of said at least two polypeptides into a multimeric complex can therefore result in various complexes, each with a different subunit composition. In theory, two types of complexes can be formed: homomeric complexes comprising only unaltered X subunits or only fused Y subunits, and heteromeric, or chimeric, complexes comprising a mixture of X and Y subunits. Depending on the number of X and Y subunits present in a complex, various types of chimeric complexes are possible. For example, a trimeric complex may comprise two X subunits and one Y subunit or vice versa; a tetrameric complex may comprise three, two or only one X subunit and one, two or three Y subunits, respectively; and so onAs will be understood, a protein complex of the invention only relates to the latter (chimeric) protein complexes; a homomeric X complex lacks a molecule of interest and a homomeric Y complex, if assembled at all, is probably not capable of binding to a receptor because of the presence of steric hindrance by the large number of (bulky) fused molecules of interest. Furthermore, a homomeric protein complex wherein all subunits are fused to an antigen, may not be a useful carrier molecule for multimeric antigens because of a sub-optimal orientation/ conformation of the individual antigen monomers with respect to immunogeinc properties. Thus, a chimeric protein complex of the invention needs to be isolated from a mixture of chimeric and homomeric complexes.

Because a fused subunit is by definition larger than an unaltered subunit, complexes with different subunit compositions will have different sizes. This difference allows for the isolation of the desired chimeric complexes from a mixture of homomeric and chimeric protein complexes according to the size of the complexes. In a preferred embodiment, a protein complex of the invention is isolated using gel filtration chromatography. Gel filtration chromatography (also known as size-exclusion chromatography or molecular sieve chromatography) can be used to separate proteins according to their size. Standard information regarding protein chromatography can be obtained from handbooks used in the field, such as "Protein Purification: Principles and Practice" by RK Scopes (Springer-Verlag 3rd edition, January 1994; ISBN 0387940723).

Briefly, during gel filtration, a (mixture of) proteins in solution is passed through a column that is packed with semipermeable porous resin. The semipermeable resin has a range of pore sizes that determines the size of proteins that can be separated with the column. This is called the fractionation range or exclusion range of the resin. Proteins larger than the exclusion range of the resin are unable to enter the pores and pass quickly through the column in the spaces between the resin. This is known as the void volume of the column. Small proteins and other low molecular weight substances that are below the exclusion range of the resin enter all the pores in the resin and their movement through the column is slowed because they must pass through the entire volume of the column. Proteins of a size that falls within the exclusion range of the column will enter only a portion of the pores. The movement of these proteins will be slowed according to their size; smaller proteins will move through the column more slowly because they must pass through a larger volume. To separate a protein sample by gel filtration chromatography, the column must first be equilibrated with the desired buffer. This is accomplished by simply passing several column volumes of the buffer through the column. Equilibration is an important step because the equilibration buffer is the buffer in which the protein sample will elute. Next, the sample is loaded onto the column and allowed to enter the resin. Then more of the equilibration buffer is passed through the column to separate the sample and elute it from the column. Fractions are collected as the sample elutes from the column. Larger proteins elute in the early fractions and smaller proteins elute in subsequent fractions.

Gel filtration should ideally be done at cold temperatures because in addition to reducing degradation of the protein complex it also helps reduce diffusion of the sample during the run, which improves resolution. Separation of proteins is enhanced by using a longer column but the longer running time can increase degradation of the protein.

The choice of a chromatography medium is an important consideration in gel filtration chromatography. Some common gel filtration chromatography media are: Sephadex G-50 (suitable for fractionation of proteins in the range of 1-30 kD); Sephadex G-75; Sephadex G-100 (4-150 kD); Sephadex G-200 (5-600 kD); Bio-Gel P-10 (1.5-20 kD); Bio-Gel P-30 (2.4-40 kD); Bio-Gel P-100 (5-100 kD) and Bio-Gel P-300 (60-400 kD). Sephadex is a trademark of Pharmacia. Bio-Gel is a trademark of Bio-Rad. The best separation occurs for molecules eluting at about 0.6 column volume, but the peaks typically get broader the later they come off. In one embodiment, a wide fractionation range material is used to give an initial cut, eliminating much larger and much smaller proteins, and subsequently a narrower range material is used for best separation. A gel filtration column can be calibrated using standards (proteins) of known molecular weights. A calibration curve can be constructed showing the retention times as a function of the log MW (logarithm of molecular weight). In a method of the invention, homomeric protein complexes are advantageously used to indicate the range wherein the heteromeric complex will elute from the column. This will be explained further in the following example, which elaborates on one of the examples mentioned above. A host cell (referred to as "XY") is provided which expresses subunits X and Y, both subunits being capable of forming a tetramer with itself and/or each other. Furthermore, a host cell "X" only expressing subunit X (an unaltered subunit) and a host cell "Y" only expressing subunit Y (a subunit fused to a molecule of interest) are provided. A suitable gel filtration medium is selected according to the size of the predicted size of the homomeric X and Y complexes (four times the size of subunits X and Y, respectively). By definition, chimeric protein complexes of the invention will have a size larger than the X homomer and smaller than the Y homomer. Accordingly, the elution volume of all chimeric protein complexes (XYYY; XXYY; and XXXY) will be larger than that of the Y homotetramer (being the largest complex) but smaller than that of the X homotetramer (being the smallest complex). Application of a protein sample of host cell "Y" containing only Y homotetramers and a protein sample of host cell "X" containing only X homotetramers to a gel filtration column can easily reveal the "boundaries" of the elution volume of chimeric proteins produced by host cell "XY". For example, column fractions are collected and analysed for the presence of X or Y subunits by SDS-PAGE followed by Western blotting using specific reagents e.g. antibodies. If desired, conditions (column size, column diameter, flow rate etc.) can be adjusted to optimize the resolution of X tetramers from Y tetramers. Of course, the larger the size difference between X and Y (i.e. the larger the molecule of interest), the more easy it will be to resolve different tetrameric complexes from each other. However, the invention specifically relates to solving problems caused by large size differences (e.g. complexes with large antigens). Therefore, separation of complexes according to the invention should not cause major problems. Once the elution volume of chimeric complexes is determined, a protein sample of host cell "XY" can be applied to the column and eluted from the column under the same conditions as were used for the calibration with homotetramers. Column fractions are collected and analysed for the presence of X and/or Y subunits as described above. SDS-PAGE of non-boiled protein fraction can be performed to analyze the size of the intact protein complex. Fractions containing a chimeric complex of the invention while being devoid of a homomeric complex are saved for further use. Fractions can either be pooled together to yield a mixture of protein complexes of the invention. Fractions can also be kept apart e.g. to yield the complexes XYYY, XXYY and XXXY as separately isolated complexes.

Conventional gel filtration chromatography can be a time consuming process. The procedure can be significantly speed up is the particle size of the chromatography medium or resin is reduced and the column is made smaller. This requires special equipment using higher pressure to get the liquid to flow through the column. In a preferred embodiment, a protein complex of the invention is isolated using high pressure liquid chromatography (HPLC) or fast performance liquid chromatography (FPLC).

In a method of the invention, an isolated protein complex is further characterized to determine its capacity to bind to a cell surface receptor or to a molecule mimicking the receptor binding moiety, e.g. D-galactose mimicking the GM1 receptor can be used for characterizing (or purifying) an LTB- or CTB-based protein complex of the invention. In a preferred embodiment, binding comprises permanent binding since this can be detected more easily than transient binding. Isolated or purified receptors can be used but also cells expressing a receptor, or membrane material derived of these cells, may be used. A protein complex of the invention is contacted with a receptor under conditions that are suitable for binding of the complex to its receptor. Conditions that may influence receptor binding include ionic strength (pH; salts) and temperature. Thereafter, the amount of bound complex is determined. In a preferred embodiment, binding of an isolated protein complex of the invention to a cell surface receptor is performed by an enzyme-linked immunosorbent assay (ELISA) or a procedure essentially based thereon. The basic principle of an ELISA is to use an enzyme to detect the binding of an antigen (Ag) to an antibody (Ab). The enzyme converts a colorless substrate (chromogen) to a colored product, indicating the presence of Ag:Ab binding. In a specific embodiment of the invention, a protein complex is based on B subunits of the heat labile enterotoxin (LT) of *E*. *coli. In vivo,* pentamerization of B subunits and binding of the pentamer to its natural receptor GM1 is the key event leading to uptake of the toxin and ultimately triggers the immonumodulatory events associated with mucosal immunity. As is exemplified herein, binding of a protein complex of the invention that is based on LTB subunits, is easily determined using GM1-ELISA as described by De Haan et al (Vaccine 1996; 1:777-783). By using such type of an assay, it will be clear that an LTB-based protein complex of the invention, comprising at least one unaltered LTB subunit and at least one LTB-fusion protein, has retained its native pentamer formation.

In a further aspect of the invention, a host cell comprising a protein complex of the invention is provided. As mentioned above, a cell is preferably an edible cell comprising a protein complex capable of delivering one or more antigens to and across mucosal surfaces. Such a complex is advantageously used as a mucosal carrier molecule. Accordingly, an edible cell, or an extract thereof, comprising a mucosal carrier molecule of the invention can be used for oral vaccination e.g. via food or drinking water.

As said earlier, conventional uses of carrier molecules are essentially limited to the delivery of relatively small antigens. However, since a chimeric complex according to the invention has retained its ability to bind to a cell surface receptor, virtually irrespective of the size of the fused protein, it is now possible to use carrier molecules for the delivery of relatively large molecules of interest (antigens etc). Furthermore, a (plant-based) vaccine comprising a protein complex or a cell according to the invention is provided, as well as a pharmaceutical composition comprising an effective amount of said vaccine. A vaccine of the invention can be a multicomponent vaccine comprising a protein complex of the invention comprising at least two different antigens.

In yet another aspect of the invention, a method is provided for increasing an immune response of a subject to a specific pathogen which comprises orally administering to the subject at least one dose of an effective amount of a protein complex of the invention, wherein the molecule of interest is an antigen. This method also opens the way to deliver protective antigens, including large (structural) antigens, to the immune system located in the intestinal tract upon oral delivery of the complex through feeding host (plant) cells or host cell compounds. Also provided herein is a method for mucosal (nasal, rectal or vaginal) immunisation comprising the administration of a vaccine of the invention to a subject via the preferred route immunization route. The invention thus provides an expansion of the size and variety of antigenic compounds that can be incorporated into carrier molecule-based vaccines

### LEGENDS

Figure 1
   Representation of the T-DNA part of the binary vectors pLANTIGEN4, 12, 13 and 15 containing the different LTB vaccine gene constructs. LB, left T-DNA border sequence; PNOS nopaline synthase promoter; NPTII, neomycin phosphotransferase II gene, selectable kanamycin resistance marker; TNOS, nopaline synthase terminator sequence; PPAT, class I patatin promoter; RB, right T-DNA border sequence; SP, signal peptide; synLT-B, synthetic gene for LTB; B, unique BamHI site for cloning; KDEL, endoplasmic reticulum retention signal; CPV, canine parvo virus epitope; Ala, alanine; HA, influenza virus hemagglutinin epitope; CSFV E2, classical swine fever virus E2 glycoprotein lacking transmembrane domain.
Figure 2
   Western analysis of tuber extracts (25 microgram total protein each) using LTB5 conformational monoclonal antibody VD12 (A) and CSFV E2 conformational mAb V3 (B). Lane 1, pL(4+13)-16; lane 2, protein size marker; lane 3, control extract; lane 4, pL1331; lane 5, pL1317 and lane 6, pL417. Arrows left in A indicate LTB5 containing pentameric complexes and in B, CSFV E2 conformational epitope containing complex.

### EXAMPLES

### Example 1: Construction of LTB subunit vaccine expression cassettes

A schematic overview of the T-DNA part of the binary plant expression vector pBINPLUS (Van Engelen et al., 1995) and the gene inserts of all the pLANTIGEN vaccine constructs reported here, is represented in Figure 1. All genes were placed under control of the class I patatin promoter (Ppat) for expression in tubers only and in addition harbour a DNA sequence that codes for a KDEL sequence at the C-terminus of the respective fusion proteins for retention in the ER (Munro and Pelham, 1987).
pL4: The design and construction of a synthetic gene for LTB (synLT-B) and the generation of the binary plant expression vector pLANTIGEN4 (pL4) was described before (Lauterslager et al., 2001). pL4 harbours the synthetic gene for LTB (synLT-B) with a unique BamHI restriction site just after the sequence coding for the mature LTB protein and preceding the sequence coding for KDEL. All synthetic sequences were made in such a way and cloned in this unique site, that all were in frame with LTB and the KDEL sequence at the carboxy terminus.
pL12: The core of the fragment coding for the CPV epitope cloned in pLANTIGEN12 (pL12), codes for the amino acid sequence SDGAVQPDGGQPAVRNERAT (Langeveld et al., 1994). pLANTIGEN12 was made by cloning a synthetic BamHI/BglII fragment coding for the amino-terminal region of the viral protein VP2 of canine parvovirus (CPV) into the unique BamHI site of pL4. The synthetic fragment was made by ligation of fragments derived from oligo's as described before (Florack et al., 1994). Oligo's were from Eurogentec (Belgium).
pL13: In pLANTIGEN13 (pL13) a fragment coding for the CSFV E2 glycoprotein lacking the C-terminal transmembrane (TM) region was ligated. In wildtype CSFV the E2 glycoprotein is transmembrane bound. pL13 was constructed by cloning a BamHI fragment coding for the E2 mature protein of the classical swine fever virus (CSFV) into the unique BamHI site of pL4. The fragment coding for CSFV E2 was obtained by PCR of pPRb2 (Hulst et al., 1993) using oligos 5'-gttcatccttttcactgaattctgcg-3' and 5'-cgcagaattcagtgaaaaggatgaac-3'. ).
pL15: In pLANTIGEN15 (pL15), the CPV sequence was cloned twice together with a doubled HA epitope sequence, each separated by two alanine residues for spacing. The HA epitope codes for the decapeptide FERFEIFPKE and represents amino acids 111-120 of PR8 HA-1 (Hackett et al., 1985). CPV is a linear B cell epitope whereas HA is T cell specific. pL15 was constructed by cloning a synthetic fragment coding for a tetrameric sequence consisting of a doubled decapeptide of influenza virus hemagglutinin (HA) heavy chain together with a doubled CPV epitope similar to what was cloned in pL12, into the unique BamHI site of pL4. All four epitope sequences were cloned in such a way that they were separated by two alanine residues each.

### Example 2: Host cell transformation, growth and protein extraction

Previously we have described the generation of 22 independent transgenic potato plants containing the pL4 gene constructs which resulted in 16 lots of tubers (Lauterslager et al., 2001). Since then, more transformation experiments were conducted which yielded additional 31 independent tuber lots harbouring the pL4 gene construct.
In the present invention, binary expression vectors described in Example 1, and combinations thereof, were introduced in *Agrobacterium tumefaciens* strain Ag10 (Lazo et al., 1991) by electroporation and used for transformation of *Solanum tuberosum* cultivar Désirée (De Z.P.C., Leeuwarden, The Netherlands). Transformation, growth, selection of transgenic shoots and tuber production are described previously (Lauterslager et al., 2001). Transformation of stem internodes of potato cultivar Désirée with pL12 generated 27 independent transgenic plants of which 22 produced tubers in the greenhouse. Transformation with pL13 generated 23 plants of which 20 formed tubers and transformation with pL15 yielded 31 plants of which 20 produced tubers.

Typically, 200 to 300 grams of tubers were harvested after 2-4 months from greenhouse grown plants. Extracts were made from freshly harvested tuber material of approximately the same size to reduce effects caused by storage or tuber age. For the isolation of a protein complex produced by the potato host cell, freshly harvested tubers of approximately 5 cm diameter were used. Skinless tuber slices were extracted in 25 mM sodium phosphate pH 6.6, 100 mM NaCl, 1 mM ethylenediamine tetraacetic acid (EDTA), 50 mM sodium ascorbate, 1% Triton X-100 and 20 mM sodium metabisulphite. Tissue homogenate was centrifuged at 4°C, 12000 rpm for 5 min and supernatant was collected and transferred to a fresh tube. Total soluble protein was estimated by the method of Bradford (Bradford, 1976) using bovine serum albumin (BSA) as standard.

Expression or accumulation of LTB pentameric complexes was confirmed by Gm1-ELISA.

### Example 3: Determining Receptor binding

The amount of functional chimeric protein complexes isolated from transgenic potato tubers was estimated by a modified ganglioside GM1 enzyme-linked immunosorbent assay (GM1-ELISA). Microtiter plates (PolySorp Immunoplates, Nunc) were coated with monosialoganglioside GM1 from bovine brain (Sigma Aldrich, St. Louis, USA) at 5 µg/mL in phosphate buffered saline (PBS). Fixed amounts, typically 5 µg of total soluble, extractable tuber protein, were loaded onto coated plates, pretreated by washing three times with deionized water and blocked for 1 h at room temperature with 2% skimmed milk, 0.1% bovine serum albumin and 0.1% Tween-20 in PBS under continuous shaking at 100 rpm. A serial twofold dilution of recombinant LTB produced by an *Escherichia coli* expression vector was added to each plate spiked with equal amounts of total protein from pBINPLUSPAT tubers. Binding was allowed for 16 h at 4°C. After washing plates three times with deionized water, plates were incubated with monoclonal antibody VD 12, specific for LTB pentamers, for 1 h at room temperature in a 1: 1000 dilution. After rinsing with deionized water three times, plates were further incubated with AP-labelled sheep-anti-mouse antibody and bound label was detected with 4-nitrophenylphosphate (disodium salt hexahydrate; Janssen Chimica). Detection was at 405 nm in a Bio-Rad Benchmark Microplate Reader (Bio Rad, Veenendaal, The Netherlands) using Microplate Manager/PC version 4.0 software for standard curve analysis, calculation of concentrations and standard deviation. The amount of pentameric LTB was estimated by comparison of the readings of samples comprising isolated pentamers with the results from the standard curve. Samples were analysed at least twice in independent experiments. For the calculation of molar rates, the amount of total protein per gram fresh weight of tuber was set at 7 mg/g. The molar weight of the fusion proteins was deduced from the known amino acid sequences of the mature proteins encoded by the respective pLANTIGEN fusion constructs.

### Example 4: Chimeric vaccine composed out of LTB and LTB-CSFV E2

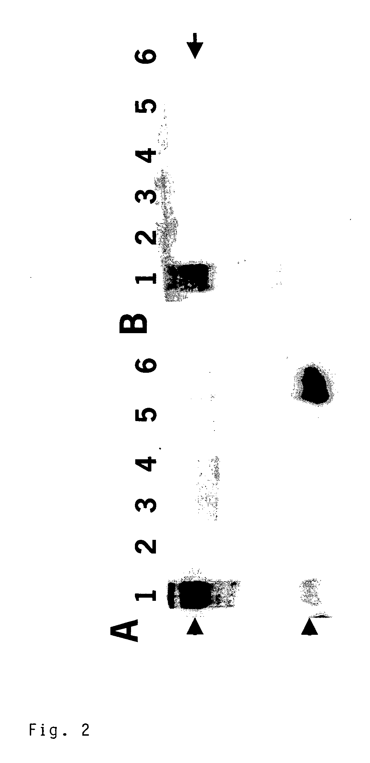

Binary expression vectors pL4 (Lauterslager et al., 2001) and pL13 (LTB-CSFV E2 fusion; for overview see Example 1) were introduced in potato by co-transformation using *Agrobacterium tumefaciens* mediated transformation of *Solanum tuberosum* cv. Désirée (De Z.P.C., Leeuwarden, The Netherlands) essentially as described before (Lauterslager et al., 2001) and outlined above (Example 1). To enable co-transformation, prior to infection of stem internodes both recombinant *Agrobacteria* harbouring pL4 and pL13 were mixed in a 1:1 ratio at OD₅₉₅= 1. The mixed bacterial suspension was used in transformation experiment and regeneration of transformed cells and selection of transgenic shoots was as described before. Seventy-one (71) independent transgenic shoots were selected and analysed for the presence of pL4 and/or pL13 gene constructs to reveal co-transformed events which were selected for further analysis. To this end, genomic DNA was isolated from leaf material collected of individual plants and submitted to PCR using specific primers that can distinguish pL4 and pL13 gene construct. Both gene constructs are under the control of a Patatin promoter and nopaline synthase terminator sequence (Lauterslager et al., 2001), and are identical at the amino terminal part of the coding region comprising the synthetic LTB gene (see Example 1). Primer 1, LTB11, 5'-ggtgatcatcacattcaagagcggtgaaacatttcaagtc-3', which was used in the construction of the synthetic gene for LTB is located in said identical part and is approximately 40 basepairs upstream of the unique *Bam*HI site into which the coding sequence for the mature part of CSFV E2 glycoprotein was cloned to construct a LTB-CSFV E2 genetic fusion as present in pL13 (see Figure 1). Primer 2, Tnosminus50, 5'-atgataatcatcgcaagaccg-3', is part of the nopaline synthase terminator sequence common to both expression cassettes and is located approximately 50 basepairs downstream of the stopcodons of pL4 and pL13 gene constructs. Amplification conditions were: 40 cycles, each 94°C for 30 seconds to enable denaturation, annealing at 56°C for 45 seconds followed by elongation at 68°C for 2 minutes, using AccuTaq polymerase (Sigma-Aldrich) at optimal conditions according to the manufacturer. PCR reaction mixtures were submitted to 1.2% agarose gel electrophoresis in 0.5 X TBE and gels were scanned for the presence of fragments corresponding to the amplified gene constructs of pL4 and/or pL13 for which the predicted sizes are well known and were deduced from their known gene sequences and the use of primers LTB11 and Tnosminus50. Plants that were positive for both gene constructs and hence were transgenic for pL4 and pL13 expression cassettes (co-transformed) were transferred to the greenhouse and grown to maturity for the production of tuber material for further analysis of accumulation of chimeric vaccine. Tubers were harvested and the amount of Gm1-binding LTB pentamers was evaluated as described in Example 2 and 3 using an anti-LTB5 conformational monoclonal antibody VD12. Several of the plants showed significant accumulation of Gm1-binding LTB pentamers and were further characterized. Tuber material of one selected co-transformed potato plant that accumulated LTB5 pentamers as deduced from binding to ganglioside Gm1, pL(4+13)-16 at approximately eight (8) microgram per gram fresh weight (FW) tuber, was further evaluated by Western blotting using either VD12, anti-LTB5 conformational monoclonal antibody (Figure 2A) or V3, anti-CSFV E2 conformational monoclonal antibody (Figure 2B). To this end, twenty-five (25) micrograms of total protein of tuber extracts of said plant and others was loaded onto a 10% SDS-PAGE gel and run under semi-native, nonreducing conditions to enable separation of protein complexes. Following semi-native SDS-PAGE, separated proteins were blotted onto nitrocellulose using standard techniques in CAPS buffer for subsequent Western analyses using specific antibodies. Specific binding/recognition was visualized using a horseradish peroxidase labelled secondary antibody and LumiLight substrate and visualized in a LumiImager (Roche, Boehringer, Germany). As controls pL1317 and 1331 were used, both harbouring pL13 gene construct. PL1317 was previously identified as the highest expressor of Gm1-binding pentamers for LTB-CSFV E2 fusion protein amongst twenty independent pL13 transgenic plants. Furthermore pL417 harbouring pL4 gene construct and accumulating approx. 17 microgram/g FW Gm1-binding LTB pentamers and control negative for Gm1-binding pentamers were used.

Figure 2A visualizes the results of the analysis of 25 microgram amounts of total protein extracts of these plants using the conformational anti-LTB5 monoclonal antibody VD 12 and Figure 2B upon analysis with V3, specific for conformational CSFV E2. Loaded were co-transformed plant pL(4+13)-16 (lane 1; co-transformed with pL4 and pL13), protein size marker (lane 2), a control extract (lane 3), extracts of pL1331 (lane 4) and 1317 (lane 5), both harbouring the LTB-CSFV E2 gene fusion only, and pL417 (lane 6) transformed with pL4 and accumulating "unmodified" rec-LTB only.

From Figure 2 it is apparent that pL(4+13)-16 accumulates a significant amount of protein structures that are significantly larger in size than homo pentameric LTB5 (compare lane 1 and 6) and recognized by both conformation specific monoclonal antibodies VD12 and V3 (compare lane 1 in A and B) proving the existence of a chimeric complex that harbours both pentameric LTB5 structures recognized by VD 12 and at the same time the antigenic CSFV E2 dimeric epitope recognized by V3. The latter is nearly absent in pL13 which is comprised of a homo pentameric LTB-CSFV E2 fusion protein. In addition, the approx. 300 kDa homopentameric LTB-CSFV E2 complex is nearly visible after analysis with VD12 and V3 which can be explained by its very low expression level as observed before. From Figure 2 and Gm1 ELISA it is also apparent that only a chimeric vaccine comprising both LTB (pL4) and LTB-CSFV E2 (pL13), as present in pL(4+13)-16 (Figure 2A and B, lane 1), facilitates the accumulation of a significant amount (8 micrograms per gram FW tuber) of a molecule that harbours both Gm1-binding properties and an antigenic CSFV E2 epitope whereas a molecule comprising only LTB-CSFV E2 gene fusion as present in pL1317 cannot (compare lanes 1 and 4 in Figure 2A and B).

### References

- Arakawa, T., Chong, D.K.X., Langridge, W.H.R. (1998) Efficacy of a food plant-based oral cholera toxin B subunit vaccine. Nature biotechnology 16: 292-297.
- Arakawa, T., Chong, D.K.X., Merrit, J.L., Langridge, W.H.R. (1997) Expression of cholera toxin B subunit oligomers in transgenic potato plants. Transgenic Research 6: 403-413.
- Bakker, H., Bardor, M., Molthoff, J.W., Gomord, V., Elbers, I., Stevens, L.H., Jordi, W., Lommen, A., Faye, L., Lerouge, P., Bosch, D. (2001) Galactose-extended glycans of antibodies produced by transgenic plants. Proceedings of the National Academy of Sciences of the USA 98: 2899-2904.
- Bowen, J.C., Nair, S.K., Reddy, R., Rouse, B.T. (1994) Cholera toxin acts as a potent adjuvant for the induction of cytotoxic T-lymphocyte responses with non-replicating antigens. Immunology 81: 338-342.
- Bradford, M.M. (1976) A rapid and sensitive method for the quantitation of microgram quantities of protein utilizing the principle of protein-dye binding. Analytical biochemistry 72: 248-254.
- Dallas, W.S., Falkow, S. (1980) Amino acid sequence homology between cholera toxin and Escherichia coli heat-labile toxin. Nature 288: 499-501.
- Dalsgaard, K., Uttenthal, Å, Jones, T.D., Xu, F., Merryweather, A., Hamilton, W.D.O., Langeveld, J.P.M., Boshuizen, R.S., Kamstrup, S., Lomonosoff, G.P., Porta, C., Vela, C., Casal, J.I., Meloen, R.H., Rodgers, P.B. (1997) Plant-derived vaccine protects animals against a viral disease. Nature Biotechnology 15: 248-252.
- Dertzbaugh, M.T., Elson, C. (1993) Comparative effectiveness of the cholera toxin B subunit and alkaline phosphatase as carriers for oral vaccines. Infection and Immunity 61: 48-55.
- Deen, N.D., Van Holten, C., Claassen, E., Boersma, W.J.A. (1993) Peptide-induced memory (IgG) response, cross-reactive with native proteins, requires covalent linkage of a specific B cell epitope with a T cell epitope. European Journal Immunology 23: 630-634.
- Engelen, F.A. van, Molthoff, J.W., Conner, A.J., Nap, J.-P., Pereira, A., Stiekema, W.J. (1995) pBINPLUS: an improved plant transformation vector based on pBIN19. Transgenic Research 4: 288-290.
- Florack, D.E.A., Dirkse, W.G., Visser, B., Heidekamp, F., Stiekema, W.J. (1994) Expression of biologically active hordothionins in tobacco. Effects of pre- and pro-sequences at the amino and carboxyl termini of the hordothionin precursor on mature protein expression and sorting. Plant molecular Biology 24: 83-96.
- Hackett, C.J., Dietzschold, B., Gerhard, W, Ghrist, B., Knorr, R., Gillessen, D., Melchers, F. (1983) The influenza virus site recognized by a murine helper T-cell specific for H1 strains: localization to nine amino acid sequence in the hemagglutinin molecule. Journal of Experimental Medicine 158: 294-
- Hackett, C.J., Hurwitz, J.L., Dietzschold, B., Gerhard, W. (1985) Synthetic decapeptide of influenza virus hemagglutinin elicits helper T cells with the same fine recognition specificities as occur in response to whole virus. Journal of immunology 135: 1391-1394.
- Haq, T.A., Mason, H.S., Clements, J.D., Arntzen, C.J. (1995) Oral immunization with a recombinant antigen produced in transgenic plants. Science 268: 714-716.
- Hein, M.B., Yeo, T.-C., Wang, F., Sturtevant, A. (1995) Expression of cholera toxin subunits in plants. Annals of the New York Academy of Sciences 792: 50-56.
- Holmgren, J. (1973) Comparison of the tissue receptors for Vibrio cholera and Escherichia coli enterotoxins by means of gangliosides and natural cholera toxoid. Infection and Immunity 8: 851-859.
- Holmgren, J., Lonnroth, I., Svennerholm, L. (1973) Tissue receptor for cholera enterotoxin: postulated structures from studies with GM1 ganglioside and related glycolipids. Infection and Immunity 8: 208-214.
- Holmgren, J., Lönnroth, 1., Mánsson, J., Svennerholm, L. (1975) Interaction of cholera toxin and membrane GM1 ganglioside of small intestine. Proc. Natl. Acad. Sci. USA 72: 2520-2524 .
- Hulst, M.M., Westra, D.F., Wensvoort, G., Moormann, R.J.M. (1993) Glycoprotein E1 of hog cholera virus expressed in insect cells protects swine from hog cholera. Journal of Virology 67: 5435-3542.
- Jagusztyn-Krynicka, E.K., Clark-Curtiss, J.E., Curtiss III, R. (1993) Escherichia coli heat-labile toxin subunit B fusions with Streptococcus sobrinus antigens expressed by Salmonella typhimurium oral vaccine strains: importance of the linker for antigenicity and biological activities of the hybrid protein. Infection and Immunity 61: 1004-1015.
- Kapusta, J., Modelska, A., Figlerowicz, M., Pniewski, T., Letellier, M., Lisowa, O., Yusibov, V., Koprowski, H., Plucienniczak, A., Legocki, A.B. (1999) A plant-derived edible vaccine against hepatitis B virus. FASEB XX: 1796-1799.
- Langridge, W.H.R. (2000) Edible vaccines. Scientific American September, 48-53.
- Lauterslager, T.G.M., Florack, D.E.A., Van der Wal, T.J., Molthoff, J.W., Langeveld, J.P.M., Bosch, D., Boersma, W.J.A., Hilgers, L.A.Th. (2001) Oral immunisation of naive and primed animals with transgenic potato tubers expressing LT-B. Vaccine 19: 2749-2755.
- Lazo, G.R., Stein, P.A., Ludwig, R.A. (1991) A DNA transformation-competent *Arabidopsis* genomic library in *Agrobacterium.* Biotechnology (NY) 9: 963-967.
- Leong, J., Vinal, A.C., Dallas, W.S. (1985) Nucleotide sequence comparison between heat-labile toxin B-subunit cistrons from Escherichia coli of human and porcine origin. Infection and Immunity 48: 73-77.
- Mason, H.S., Arntzen, C.J. (1995) Transgenic plants as vaccine production systems. TIBTECH 13: 388-392.
- Mason, H.S., Ball, J.M., Shi, J.-J., Jiang, X., Estes, M.K., Arntzen, C.J. (1996) Expression of Norwalk virus capsid protein in transgenic tobacco and potato and its oral immunogenicity in mice. Proceedings of the National Academy of Sciences of the USA 93: 5335-5340.
- Mason, H.S., Haq, T.A., clements, J.D., Arntzen, C.J. (1998) Edible vaccine protects mice against Escherichia coli heat-labile enterotoxin (LT): potatoes expressing a synthetic LT-B gene. Vaccine 16: 1336-1343.
- Mason, H.S., Lam, D.M.-K., Arntzen, C.J. (1992) Expression of hepatitis B surface antigen in transgenic plants.. Proceedings of the National Academy of Sciences of the USA 89: 11745-11749.
- Mor, T.S., Gomez-Lim, M.S., Palmer, K.E. (1998) Perspective: edible vaccines, a concept coming of age. Trends in Microbiology 6: 449-453.
- Munro, S., Pelham, H.R.B. (1987) A C-terminal signal prevents secretion of luminal ER proteins. Cell 48: 899-907.
- Richter, L., Kipp, P.B. (1999) Transgenic plants as edible vaccines. Current Topics in Microbiology and Immunology 240: 159-176.
- Sanger, F., Nicklen, S., Coulson, A.R. (1977) DNA sequencing with the chain-terminating inhibitors. Proceedings of the National Academy of Sciences of the USA 74: 5463-5467.
- Schouten, A., Roosien, J., Van Engelen, F.A., De Jong, G.A.M., Borst-Vrenssen, A.W.M., Zilverentant, J.F., Bosch, D. (1996) The C-terminal KDEI sequence increases the expression level of a single-chain antibody designed to be targeted to both the cytosol and the secretory pathway in transgenic tobacco. Plant Molecular Biology 30: 781-793.
- Spangler, B.D. (1992) Structure and function of cholera toxin and the related Escherichia coli heat-labile enterotoxin. Microbiological Reviews 56: 622-647.
- Streatfield, S.J., Jilka, J.M., Hood, E.E., Turner, D.D., Bailey, M.R., Mayor, J.M., Woodard, S.L., Beifuss, K.K., Horn, M.E., Delaney, D.E., Tizard, I.R., Howard, J.A. (2001) Plant-based vaccines: unique advantages. Vaccine 19: 2742-2748.
- Tacket, C.O., Mason, H.S. (1999) A review of oral vaccination with transgenic vegetables. Microbes and Infection 1: 777-783.
- Tacket, C.O., Mason, H.S., Losonsky, G., Clements, J.D., Levine, M.M., Arntzen, C.J. (1998) Immunogenicity in humans of a recombinant bacterial antigen delivered in a transgenic potato. Nature Medicine 4: 607-609.
- Thanavala, Y., Yang, Y.-F., Lyons, P., Mason, H.S., Arntzen, C. (1995) Immunogenicity of transgenic plant-derived hepatitis B surface antigen. Proceedings of the National Academy of Sciences of the USA 92: 3358-3361.
- Towbin, H., Staehelin, T., Gordon, J. (1979) Electrophoretic transfer of proteins from polyacrylamide gels to nitrocellulose sheets: procedures and some applications. Proceedings of the National Academy of Sciences of the USA 76: 4350-5354.

## Claims

1. A protein complex comprising at least two, preferably identical, subunits wherein at least one subunit is unaltered and at least one subunit is fused to a first molecule of interest and wherein the protein complex is able to interact with a cell surface receptor via said subunits.

2. A protein complex according to claim 1, wherein said first molecule of interest can associate with, preferably via a covalent bond, a second molecule of interest to form a multimer of interest.

3. A protein complex according to claim 1 or 2, wherein said complex is essentially based on the heat labile enterotoxin (LT) of *E. coli* or on the cholera toxin (CT) of *Vibrio cholerae,* preferably the B subunits thereof.

4. A protein complex according to any of the preceding claims, wherein said complex comprises at least two subunits provided with a molecule of interest.

5. A protein complex according to claim 4, wherein said at least two subunits are provided with a different molecule of interest.

6. A protein complex according to any one of the preceding claims, wherein said cell surface receptor is present on intestinal epithelial.

7. A protein complex according to any one of the preceding claims, wherein at least one molecule of interest is an antigen.

8. A protein complex according to claim 7, wherein said antigen is selected from the group consisting of a bacterial antigen, a viral antigen, a protozoal antigen and a fungal antigen.

9. A protein complex according to any one of claims 1 to 6, wherein at least one molecule of interest is an immunomodulatory protein, preferably a cytokine or a heat-shock protein.

10. A protein complex according to any one of the preceding claims, wherein said complex comprises five B subunits of the heat labile enterotoxin (LT) of E. *coli* or the cholera toxin (CT) of *Vibrio cholerae,* wherein at one subunit is unaltered.

11. A protein complex according to any one of the preceding claims, wherein said cell surface receptor comprises a ganglioside molecule, preferably GM1, or a mimic thereof.

12. A method for producing a protein complex according to any one of claims 1-11, comprising:
a) providing a host cell with a nucleotide sequence encoding an unaltered subunit and a nucleotide sequence encoding a molecule of interest, wherein at least one molecule of interest is fused to a subunit;
b) culturing said host cell thereby allowing expression of said nucleotide sequences and allowing for assembly of the protein complex;
c) isolating the complex;
d) determining the binding of the complex to a cell surface receptor or to a molecule which mimics a cell surface receptor.

13. A method according to claim 12, wherein said host cell is provided with said nucleotide sequences using transformation, co-transformation, crossing, re-transformation or transient transfection.

14. A cell comprising the protein complex according to any one of claims 1 to 11.

15. A cell according to claim 14, wherein said cell is a plant cell.

16. A cell according to claim 13 or 14, wherein said cell is an edible cell.

17. A composition comprising a protein complex according to any one of claims 1-11 or a cell according to any one of claims 14-16.

18. A vaccine comprising a protein complex according to any one of claims 1-10 or a cell according to any one of claims 13-15 and a pharmaceutically acceptable carrier.

19. A pharmaceutical composition comprising an effective amount of a vaccine according to claim 18.

20. Use of a protein complex according to any one of claims 1-11 as a mucosal carrier molecule.

21. A method for modulating an immune response of a subject comprising administering to the subject at least one dose of an effective amount of a protein complex according to any one of claims 1-11, wherein the molecule of interest is an antigen.

22. A method for mucosal immunisation comprising the administration of a vaccine according to claim 18 to a subject.
